(19) Europäisches Patentamt / European Patent Office / Office européen des brevets

(11) **EP 3 660 852 B1**

(12) **EUROPEAN PATENT SPECIFICATION**

(45) Date of publication and mention of the grant of the patent:
**01.05.2024 Bulletin 2024/18**

(21) Application number: **18839139.5**

(22) Date of filing: **25.07.2018**

(51) International Patent Classification (IPC):
**G16B 25/00** (2019.01)   **G16B 30/00** (2019.01)
**C07K 14/435** (2006.01)   **C12N 9/12** (2006.01)
**C12N 15/67** (2006.01)

(52) Cooperative Patent Classification (CPC):
**C12N 9/12; C07K 14/43595; C12N 15/67;
C12Y 207/11024; G16B 25/00; G16B 30/00;**
C07K 2319/43

(86) International application number:
**PCT/CN2018/097040**

(87) International publication number:
**WO 2019/020054 (31.01.2019 Gazette 2019/05)**

(54) **CODON OPTIMIZATION METHOD BASED ON IMMUNE ALGORITHM**

VERFAHREN ZUR CODON-OPTIMIERUNG AUF DER GRUNDLAGE EINES IMMUNALGORITHMUS

PROCÉDÉ D'OPTIMISATION DE CODON BASÉ SUR UN ALGORITHME IMMUNITAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.07.2017 CN 201710611752**

(43) Date of publication of application:
**03.06.2020 Bulletin 2020/23**

(73) Proprietor: **Nanjing GenScript Biotech Co., Ltd.
Nanjing, Jiangsu 211100 (CN)**

(72) Inventors:
 • **FAN, Long**
   **Nanjing, Jiangsu 211100 (CN)**
 • **SUN, Yan**
   **Nanjing, Jiangsu 211100 (CN)**
 • **WU, Dongming**
   **Nanjing, Jiangsu 211100 (CN)**
 • **HUANG, Xiaoluo**
   **Nanjing, Jiangsu 211100 (CN)**
 • **ZHANG, Lihua**
   **Nanjing, Jiangsu 211100 (CN)**
 • **LIU, Zhenyu**
   **Nanjing, Jiangsu 211100 (CN)**

(74) Representative: **Wichmann, Hendrik
Wuesthoff & Wuesthoff
Patentanwälte und Rechtsanwalt PartG mbB
Schweigerstraße 2
81541 München (DE)**

(56) References cited:
   **CN-A- 101 490 262      CN-A- 106 951 726
   US-A1- 2014 244 228**

• JU XIN CHIN ET AL: "Codon Optimization OnLine (COOL): a web-based multi-objective optimization platform for synthetic gene design", BIOINFORMATICS, vol. 30, no. 15, 10 April 2014 (2014-04-10), pages 2210-2212, XP055640263, GB ISSN: 1367-4803, DOI: 10.1093/bioinformatics/btu192
• LIU XINGXING ET AL: "An Improved Adaptive Immune Genetic Algorithm Based on Information Entropy", 2015 INTERNATIONAL CONFERENCE ON INDUSTRIAL INFORMATICS - COMPUTING TECHNOLOGY, INTELLIGENT TECHNOLOGY, INDUSTRIAL INFORMATION INTEGRATION, IEEE, 3 December 2015 (2015-12-03), pages 6-9, XP032843514, DOI: 10.1109/ICIICII.2015.89 [retrieved on 2016-01-06]

**(Cont. next page)**

- ZHANG R ET AL: "Optimization of DNA encoding sequence based on immune adaptive genetic algorithm", JOURNAL OF INFORMATION AND COMPUTATIONAL SCIENCE, SUN YAT-SEN (ZHONGSHAN) UNIVERSITY, CN, vol. 5, no. 1, 31 December 2007 (2007-12-31), pages 103-110, XP009526555, ISSN: 1548-7741
- Zheng, Jiangang et al.: "DNA-Immune-Genetic algorithm based on information entropy", Computer Simulation, vol. 23, no. 6, 30 June 2006 (2006-06-30), pages 163-165, XP009519109,
- Fu, Ping et al.: "Clustering analysis of immune-genetic algorithm based on information entropy", Computer Engineering, vol. 34, no. 6, 31 March 2008 (2008-03-31) , pages 227-228,232, XP009519108, ISSN: 1000-3428
- Sandhu,K.S. et al.: "GASCO: Genetic algorithm simulation for codon optimization", In Silico Biology, vol. 8, no. 2, 13 April 2008 (2008-04-13), pages 187-191, XP009189299, ISSN: 1386-6338

Remarks:
The complete document including Reference Table(s) and the Sequence Listing(s) can be downloaded from the EPO website

**Description**

**TECHNICAL FIELD**

The present invention relates to a protein engineering technology, and in particular to a codon optimization method in protein engineering which is, a computer-implemented codon optimization method based on an immune algorithm.

**BACKGROUND**

Codon degeneracy refers to the phenomenon that an amino acid can be encoded by multiple different codons during protein translation. The different codons encoding the same amino acid are called synonymous codons. A protein consisting of 200 amino acids in length can generally be encoded by more than $10^{20}$ different DNA sequences. In different species, the occurrence frequency of the synonymous codons is different, and such a phenomenon is called codon preference. Codon optimization is mainly based on factors such as the codon preference of a host expression system. On the premise of not changing the amino acid sequence of a protein, a computer algorithm is used to screen out the DNA sequence that can express the protein most efficiently in the host expression system from a large number of DNA coding sequences.

At present, the main factors that are often considered as affecting protein expression in the process of codon optimization include the codon preference of a host cell (commonly used characterization parameters thereof include a codon adaptation index [CAI], a duplex codon preference of a host cell [Codon Context], CBI [Codon Bias Index], ENC [Effective Number of Codon], FOP [Frequency of Optimal Codons], CPP [Codon Preference Parameter], and tAI [tRNA adaptation index]), the number of Hidden Stop Codons, a GC content, a rare codon content, the number of mRNA inhibitory regulatory motif, a mRNA secondary structure (mainly including a hairpin structure and minimum free energy), scoring of key codons and mathematical models in machine learning, a microRNA binding site, a G4 content, and a codon preference of a protein secondary structure (Joshua B. Plotkin & Grzegorz Kudla, Nature Reviews Genetics, 2011). The software and algorithms currently available for codon optimization include DNAWorks, Jcat, Synthetic gene designer, GeneDesign 2.0, OPTIMIZER, Eugene, mRNA Optimizer, COOL, D-Tailor, UpGene, GASCO, Codon Harmonization, QPSO, GeMS and ATGME (Evelina Angov, Biotechnology Journal, 2011; Nathan Gould et al., Frontiers in Bioengineering and Biotechnology, 2014). A method of optimizing a nucleotide coding sequence for expression in a predetermined host cell is described in US 2014/244228 A1.

Compared with a heuristic algorithm (such as a particle swarm and genetic algorithm) that has been used in codon optimization algorithms, an immune algorithm has its unique advantages. The immune algorithm is an improved genetic algorithm based on a biological immune mechanism. It enables the objective function of an actual problem to be solved to correspond to an antigen and enables the solution of the problem to correspond to an antibody. According to the principle of biological immunity, it can be seen that a biological immune system automatically generates corresponding antibodies through cell division and differentiation to resist antigens that invade living organisms. Such a process is referred to as immune response. In the process of immune response, some antibodies are preserved as memory cells, and when antigens of the same type invade again, the memory cells are activated and produce a large number of antibodies rapidly, which makes the re-response faster and stronger than the initial response, which reflects the memory function of the immune system. After binding with antigens, the antibodies destroy the antigens through a series of reactions. At the same time, different antibodies also promote and inhibit each other to maintain the diversity of antibodies and an immune balance. Such a balance is achieved according to a concentration mechanism, that is, the higher the concentration of antibodies, the more inhibited the antibodies are; and the lower the concentration, the more promoted the antibodies are, reflecting the self-regulation function of the immune system.

**SUMMARY**

An objective of the present invention is to solve the problems of long cycle and poor expression accuracy of existing codon optimization methods, and to invent a codon optimization method based on an immune algorithm, which can effectively complete large-scale search of a codon optimization space within a limited time, i.e., screening out the DNA sequence that has the most effective expression from a protein coding sequence set.

The technical solution of the present invention is the computer-implemented codon optimization method defined in the claims.

The codon optimization method is based on an immune algorithm and includes that an immune algorithm and a genetic algorithm are successively used to respectively perform local multi-objective optimization and global multi-objective optimization on a protein coding sequence, and then an exhaustive method is used to perform fine adjustment and optimization on the sequence, so as to search the optimal expression sequence to the greatest extent.

In particular, the method of the present invention includes the following three steps: a first step of local optimization, that is, cleaving the protein sequence into nonoverlapping sequence fragments $A_1, A_2...A_n$, and then using the immune algorithm to complete the codon optimization for each sequence fragment, so as to generate an approximately optimal DNA sequence set $B_1, B_2...B_n$; a second step of global optimization, that is, initializing the DNA coding sequence of the full length of the protein based on $B_1, B_2...B_n$ utilizing the genetic algorithm, and screening out the optimal DNA sequence $C_1$ of the protein sequence; and a third step of fine adjustment and optimization, which comprises performing exhaustive optimization on the 5' terminal of the DNA sequence corresponding to the N-terminal region of the encoded protein to generate a DNA sequence $C_2$, and eliminating an expression inhibitory motif, to finally generate the optimal expression sequence D.

The protein refers to a compound consisting of more than 20 amino acids. Suitable proteins include secretory proteins, membrane proteins, cytoplasmic proteins, nuclear proteins, etc. in terms of localization; antibody proteins, regulatory proteins, structural proteins, etc. in terms of function; homologously expressed proteins and heterologously expressed proteins in terms of source; natural proteins and artificially-modified proteins, complete proteins/antibodies and truncated partial proteins/antibodies, and fusion proteins formed from 2 or more proteins or from a protein and a peptide chain in terms of sequences. Antibodies as defined in the present invention include, but are not limited to, an intact antibodies and Fabs, ScFVs, SdAb, chimeric antibodies, bispecific antibodies, and Fc fusion proteins.

The immune genetic algorithm adopts a multi-objective optimization method to perform local optimization on the protein fragments, the population initialization is based on a duplex codon table of a sequence encoding a highly-expressed protein, and each gene is directly encoded by synonymous codons; and in the optimization process, antibody diversity is ensured and the phenomenon of population degeneration is prevented by calculating antibody information entropy, antibody population similarity, antibody concentration and polymerization fitness of the immune genetic algorithm and updating memory cells, so as to increase the global search capability of the algorithm.

The genetic algorithm adopts the multi-objective optimization method to perform global optimization on the full sequence of the protein, an initialized population is randomly generated based on optimized fragments subjected to local optimization, and each gene is directly encoded by an optimized sequence set of each protein fragment.

The fine adjustment and optimization uses the exhaustive method to calculate and sort the minimum free energy MFE, Codon Context and CAI at the 5' terminal of the DNA sequence, and selects the optimum coding sequence for the N-terminal of the protein sequence according to the sorting result.

The codon optimization method is applicable to at least one of the following host expression systems: 1) a mammalian expression system; 2) an insect expression system; 3) a yeast expression system; 4) a *Escherichia coli* expression system; 5) a *Bacillus subtilis* expression system; 6) a plant expression system, and 7) a cell-free expression system.

The codon optimization method is applicable to at least one of the following expression vectors: a transient expression vector, a stable expression vector, a viral expression vector, a non-viral expression vector, induced and non-induced expression vectors.

The beneficial effects of the present invention are as follows.

The immune algorithm is an algorithm improved from the genetic algorithm. In view of the advantage of the immune algorithm in preventing premature local convergence in optimization, the present invention is the first to introduce the immune algorithm to carry out codon optimization for local optimization, and carries out global optimization through the subsequent genetic algorithm and finally carries out fine adjustment and optimization, and thus develops a brand-new three-step hybrid optimization algorithm which combines the advantages of different algorithms; and the high efficiency of the algorithm in codon optimization is further proved by the following Examples.

Compared with the genetic algorithm, the immune algorithm of the present invention has the following characteristics: firstly, the immune algorithm has an immune memory function which can accelerate the search speed and improve the overall search capability of the genetic algorithm; secondly, it has the function of maintaining the diversity of antibodies, which can be utilized to improve the local searching ability of the genetic algorithm; and finally, it has a self-regulating function, which can be used to improve the global search ability of the genetic algorithm and avoid falling into a local solution. Therefore, the immune genetic algorithm not only retains the characteristic of random global parallel search of the genetic algorithm, but also avoids premature convergence to a comparatively great extent to ensure rapid convergence to the global optimal solution. The present invention is the first to combine the advantages of the immune algorithm and the genetic algorithm in accuracy and efficiency to carry out codon optimization through a step-by-step process (local optimization, global optimization, and fine adjustment and optimization respectively in sequence), and proves the high efficiency of the algorithm in codon optimization through example tests.

The present invention has the advantages of high speed and high efficiency.

**BRIEF DESCRIPTION OF DRAWINGS**

FIG. 1 is a schematic flow chart of an optimization algorithm of the present invention.

FIG. 2 is a schematic flow chart of an immune algorithm of the present invention (i.e., a local optimization flow).

FIG. 3 shows a flow of a genetic algorithm of the present invention (i.e., a global optimization flow).

FIG. 4 shows a flow of optimizing the 5' terminal of the DNA sequence of the present invention.

FIG. 5 is a schematic diagram of the gene sequence design of a test protein obtained by the method of the present invention.

FIG. 6 is a pTT expression vector map of the present invention.

FIG. 7 is a schematic diagram of Western Blotting results of the present invention.

## DETAILED DESCRIPTION

The following further describes the present invention with reference to the accompanying drawings and specific examples.

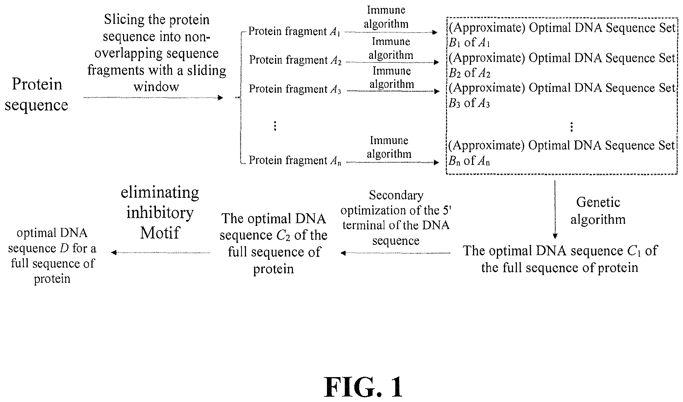

It is as shown in FIGs. 1-7.

A codon optimization method based on an immune algorithm includes that an immune algorithm and a genetic algorithm are successively used to respectively perform local multi-objective optimization and global multi-objective optimization on protein coding sequences (SEQ ID NO.3 and SEQ ID NO.4), and then an exhaustive method is used to perform fine adjustment and optimization on the sequence, so as to search the optimal expression sequences (SEQ ID NO.5 and SEQ ID NO.6) to the greatest extent, as shown in FIG. 1, wherein:

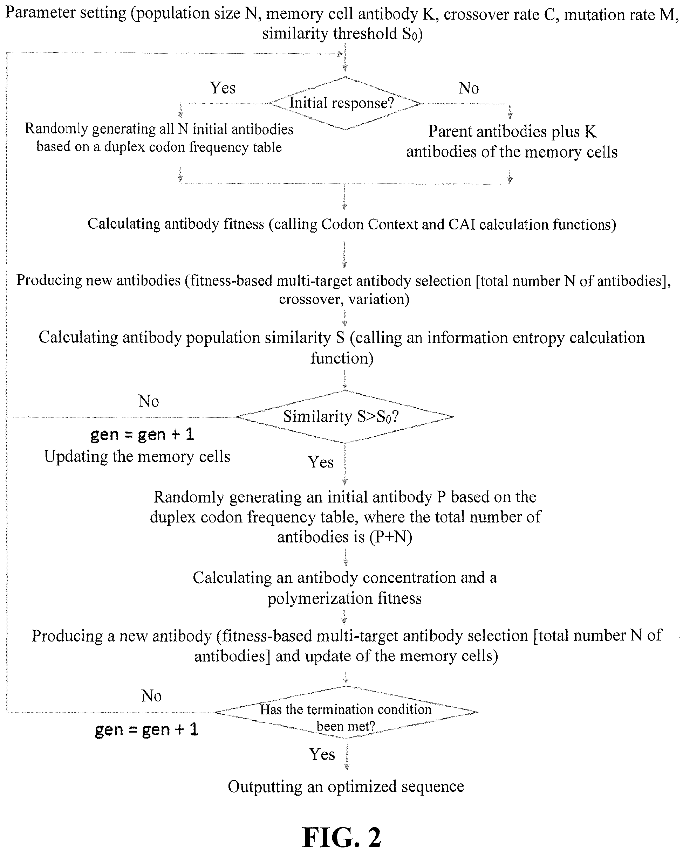

I. Immune algorithm (i.e., local optimization, see FIG. 2 for the flow).

The number of optimization variables $L$ in this step is 2, that is, two features, Codon Context and CAI, are optimized for each fragment (see below for detailed description), which belongs to multi-objective optimization. Assuming that an immune system consists of $N$ antibodies (i.e., the population size is $N$), each antibody gene has a length of $M$ (equivalent to that the number of amino acids in the protein sequence is $M$), and each gene is directly encoded with synonymous codons.

(1) According to a basic data set of different host expression systems (i.e., coding sequences of highly-expressed proteins), a codon frequency table and a duplex codon frequency table are calculated for generating sequences and calculating the codon context and CAI.

(2) In the initial response, an initial antibody is generated according to the duplex codon frequency. Taking the protein sequence $a_1a_2...a_m$ as an example, it is assumed that the synonymous codons for $a_1$ are $c_{11}$ and $c_{12}$, and the synonymous codons for $a_2$ are $c_{21}$, $c_{22}$ and $c_{23}$. The codons for the first amino acid $a_1$ are selected according to the frequencies of $c_{11}$ and $c_{12}$ in the codon frequency table. The duplex codons corresponding to the duplex amino acid $a_1a_2$ are $c_{11}c_{21}$, $c_{11}c_{22}$, $c_{11}c_{23}$, $c_{12}c_{21}$, $c_{12}c_{22}$ and $c_{12}c_{23}$, where there are two sets of duplex synonymous codons, including [$c_{11}c_{21}$, $c_{11}c_{22}$, $c_{11}c_{23}$] and [$c_{12}c_{21}$, $c_{12}c_{22}$, $c_{12}c_{23}$]. Assuming that the codon selected for $a_1$ is $C_{11}$, then the codon for the amino acid $a_2$ is selected from one of $c_{21}$, $c_{22}$ and $c_{23}$ according to the frequencies of $c_{11}c_{21}$, $c_{11}c_{22}$ and $c_{11}c_{23}$. If the codon selected for $a_1$ is $C_{12}$, then the codon for the amino acid $a_2$ is selected from one of $c_{21}$, $c_{22}$ and $c_{23}$ according to the frequencies of $c_{12}c_{21}$, $c_{12}c_{22}$ and $c_{12}c_{23}$. In brief, the selection of codons for other amino acids is related to the selection of codons for its previous amino acid and is determined by the frequency of their duplex synonymous codons, except that the codon for the first amino acid is directly selected according to the codon frequency table.

(3) In a non-initial response, the population consists of parent individuals and $K$ antibodies stored in a memory cell. The antibodies of the memory cell record the $K$ optimum antibodies that have appeared in the optimization history, where the antibodies with low fitness are gradually replaced by individuals with higher fitness in the optimization process.

(4) The fitness $F$ (including $F_{[codon\ Context]}$ and $F_{[CAI]}$) of an antibody is calculated, N progeny individuals are selected according to multi-objective optimization, and crossover and variation operations are completed for the new population. The variation here is random mutation of codon.

(5) Calculating of antibody population similarity $S$

The present invention uses Shannon's average information entropy $H(N)$ to measure the population similarity $S$.

First, $P_{ij}$ is the probability that a synonymous codon $i$ appears on an amino acid $j$, namely:

$$P_{ij} = \frac{N_{ij}}{N},$$

where $N_{ij}$ is the total number of synonymous codon $i$ that appears at the $j$-th amino acid position of all individuals in the population. Then $Hj(N)$ is the information entropy of the $j$-th gene (i.e. the $j$-th amino acid of the protein sequence), and is defined as:

$$H_j(N) = -\sum_{i=1}^{N} P_{ij} \log_2 P_{ij}$$

The average information entropy of the whole population is:

$$H(N) = -\frac{1}{M} \sum_{j=1}^{M} H_j(N)$$

The population similarity S is defined as:

$$S = \frac{1}{1 + H(N)}$$

(6) With the progress of optimization, the similarity of antibodies in the population is continuously improved. In order to avoid the homogeneity of antibodies and improve the diversity of antibodies, and thus improve the global search ability and prevent premature convergence, when the population similarity $S$ is greater than the threshold $S_0$, the metabolic function of the immune system cells is simulated to generate $P$ new antibodies, and the generation process is the same as the above (2), so that the total number of antibodies reaches $P+N$. If the population similarity $S$ is less than the threshold $S_0$, then the population continues to directly enter the next generation of evolution and the memory cells are updated.

(7) When $S>S_0$, the antibody concentration and polymerization fitness are calculated for the antibody population $P+N$. The antibody concentration refers to the percentage of antibodies similar to each antibody in the population, i.e.,

$$C_i = \frac{A_i}{N-1}$$

where $Ai$ refers to the number of antibodies whose similarity to the antibody $i$ is greater than a similarity constant $\lambda$. $\lambda$ refers to the number of identical codons among $M$ codons when two individuals are compared.

Polymerization fitness $F'$ is a value obtained after the antibody fitness $F$ is corrected according to the antibody concentration, namely:

$$F_i' = \alpha \frac{F_i}{\sum_i^N F_i} + (1-\alpha) \frac{A_i}{\sum_i^N A_i} \quad (0 < \alpha < 1)$$

According to the polymerization fitness, a progeny population is selected, and memory cells are updated, and a next round of optimization is carried out. Since we consider the two sequence features, codon context and CAI, at the same time, $F'_{[codon\ context]}$ is calculated based on $F_{[codon\ context]}$, and $F'_{[CAI]}$ is calculated based on $F_{[CAI]}$. If a termination algebra is reached, the evolution is stopped, and an optimized sequence set of a single protein fragment is output.

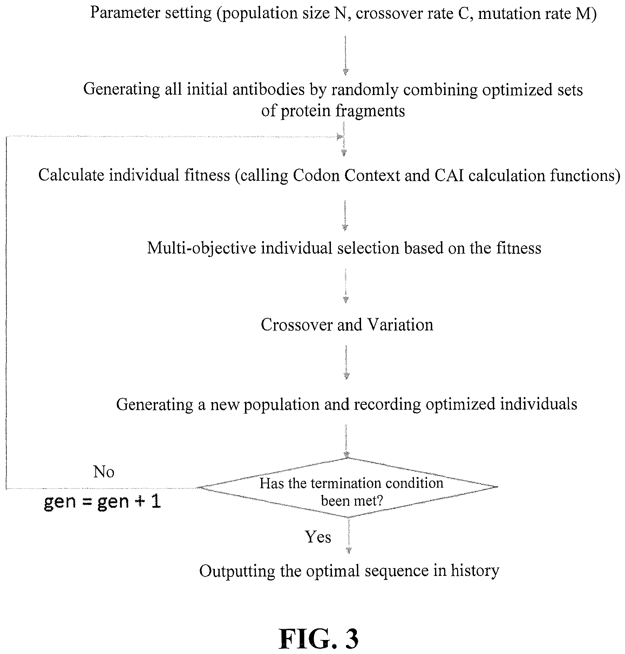

II. genetic algorithm (i.e., global optimization, see FIG. 3 for the flow).

Based on the optimized sequence set of all protein fragments generated by the optimization through the immune algorithm, an initialized population N is randomly generated. According to the flow of the genetic algorithm, fitness calculation, selection of a progeny population, crossover, variation and memory update are completed. If a termination algebra is reached, the evolution is stopped, and the optimal DNA coding sequence for the full sequence of the protein is output. The whole flow belongs to the multi-objective optimization. In the optimization process, we directly use the optimized sequence set of each protein fragment to encode each gene.

III. Fine Adjustment and Optimization.

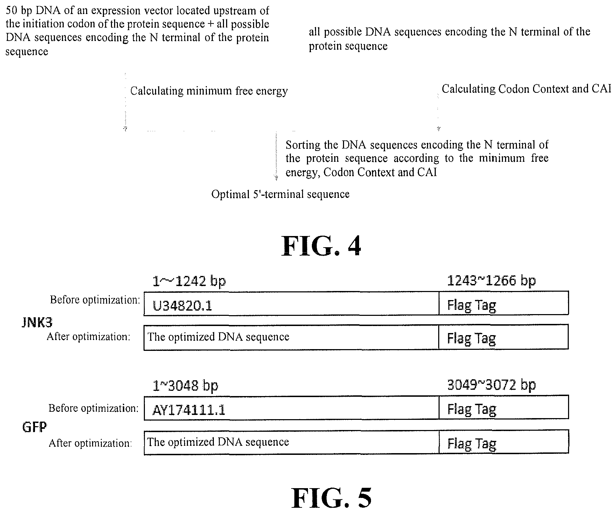

The fine adjustment and optimization consists of two steps: first, optimizing the 5' terminal of the DNA, and then eliminating the expression inhibitory motif. The optimization process of the 5' terminal of DNA is as shown in FIG. 4. The exhaustive method is used to list all possible DNA coding sequences of the N-terminal amino acid sequence (8-15 amino acids) of the protein, and to calculate their codon context and CAI. Then 50 bp (with a default value of 50 bp, and a selectable length range of 0-50 bp) of a vector sequence located upstream of an initiation codon of the protein sequence is connected to the DNA coding sequence sequentially, and the minimum free energy (MFE) of the connected sequence is calculated by software mfold. According to the minimum free energy (the greater the value, the better), the codon context (the greater the value, the better) and CAI (the greater the value, the better), the coding sequences of signal peptides are sorted to select the best 5'-terminal sequence.

IV. Details of the above flow

(1) Generation of Basic Data Set and Duplex Codon Table

The basic data set refers to highly-expressed proteins in different host expression systems and their corresponding DNA coding sequences. The duplex codon table refers to the relative fitness of all duplex codons in the basic data set (see below for the calculation method).

(2) calculation flow of codon context and CAI

a) codon relative fitness $w_{ij}$:

$$w_{ij} = \frac{x_{ij}}{x_{i\,\max}}$$

where $x_{ij}$ represents the number of the **j**-th synonymous codon of the **i**-th type of amino acid appeared in the basic data set, and $x_{imax}$ represents the number of synonymous codons with the highest use frequency for the **i**-th type of amino acid appeared in the basic data set.

b) Codon Adaptation Index (CAI) of a target sequence:

$$CAI = \left( \prod_{k=1}^{L} w_k \right)^{\frac{1}{L}}$$

where **L** refers to the number of amino acids of the target sequence (i.e., the protein sequence or fragment), $w_k$ is the codon relative fitness of the basic data set corresponding to the codon used by each amino acid codon. CAI has a value between 0 and 1. In the optimization process, we try our best to increase the CAI value of the encoding DNA.

c) Relative *fitness $p_k$* of duplex codon:

$$p_k = \frac{\alpha_{CC}^{k}}{\alpha_{AA}^{j(k)}}, \forall k \subseteq \{1,2,\ldots,3721\}$$

where there are 3,721 kinds of duplex codons (61 $\times$ 61 = 3721, without considering termination codons), $\alpha_{CC}^{k}$ represents the number of the *k*-th type of duplex codons appeared in the protein sequence basic data set or the target sequence (i.e., the protein sequence or a fragment thereof), and $\alpha_{AA}^{j(k)}$ represents the number of duplex amino acids corresponding to the duplex codons as appeared.

d) Codon Context (CC) of the target sequence:

$$CC = 1 - \frac{\sum_{k=1}^{3721}\left|p_0^k - p_1^k\right|}{3721}$$

where $p_0^k$ represents the relative fitness of the *k*-th type of duplex codons of the target sequence, and $p_1^k$ represents the relative fitness of the *k*-th type of duplex codons of the basic data set. CC has a value between 0 and 1. In the optimization process, we try our best to increase the CC value of the encoding DNA.

(3) NSGA2 and SPEA2 algorithms (NSGA2 is used by default) can be used for selection of progeny population in the multi-objective optimization process of the immune algorithm and the genetic algorithm, and two-point crossover is used for crossover.

The following further illustrates the advantages of the present invention by an example.

The host expression system used in the test is a CHO cell line, and two proteins are optimized and sequenced in total (see Table 1 for relevant information). The JNK3 protein sequence is as shown in SEQ ID NO.1, and the GFP protein sequence is as shown in SEQ ID NO.2; the coding sequences of the JNK3 protein and the GFP protein before optimization are as shown in SEQ ID NO.3 and SEQ ID NO.4 respectively, and the coding sequences of the JNK3 protein and the GFP protein after optimization are as shown in SEQ ID NO.5 and SEQ ID NO.6 respectively.

Table 1: Information of Optimized Test Protein Sequences

| Protein | GenBank accession number (Wild Type) | tag | Position of Tag |
|---------|--------------------------------------|----------|-----------------|
| JNK3 | U34820.1 | Flag tag | C terminal |
| GFP | AY174111.1 | Flag tag | C terminal |

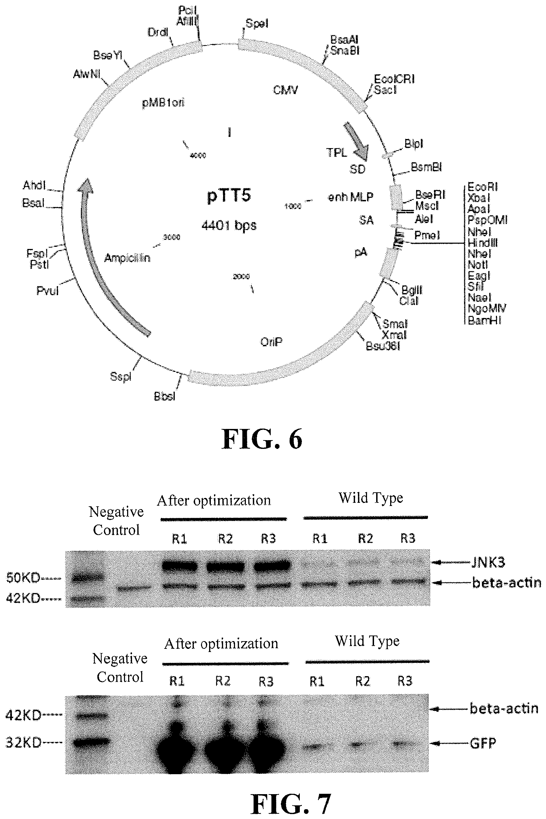

As shown in FIG. 5, the gene fragment encoding the test protein is synthesized and cloned into a pTT5 expression vector (purchased from NRC, and the plasmid map is as shown in FIG. 6) via EcoR I and Hind III cleavage sites respectively.

**Transient Expression Steps of CHO 3E7 Cells:**

1. CHO 3E7 suspension cells in the logarithmic growth phase are diluted to $5 \times 10^5$ cells /mL with a fresh FreeStyle CHO medium, and 30 mL of a cell suspension is inoculated in each 125 mL triangular flask.

2. The cells are subjected to suspension culture under conditions of 37°C and 5% $CO_2$.

3. When the cell density reaches $1\text{-}1.2 \times 10^6$ cells /mL, plasmid vectors carrying cloned target genes are transfected into CHO 3E7 cells respectively according to the dosage of 1 ug/ml by a PEI transfection reagent.

4. After 48 hours of transfection, the culture medium is centrifuged at 1500 rotations/min to harvest cells. The samples can be stored in a refrigerator at -80°C.

**Western Blot experiment steps:**

Using an anti-Flag tag antibody, the expression quantity of the target protein in a cell lysate was detected by Western Blotting. A beta-actin protein is used as internal reference. The expression experiment of each plasmid is replicated for three times. The results of Western Blotting are shown in FIG. 7.
The detailed steps are as follows.

1. CHO cells are lysed using a cell lysis buffer, and the protein concentration is determined.
2. The protein solution is added with a 5X SDS-PAGE protein loading buffer, and heated in a boiling water bath for 10 minutes.
3. The protein sample is added into sample loading wells of an SDS-PAGE gel with a micropipette, and each well is loaded with 20 ul of the sample.
4. A constant voltage electrophoresis at 140 V is used for 60 minutes, and the electrophoresis is stopped when bromophenol blue reaches near the bottom of the gel.
5. The membrane transfer voltage is 100 V, and the membrane transfer time at low temperature is 60 minutes.
6. After the membrane transfer is completed, the protein membrane is placed in a washing liquid prepared in advance, and rinsed for 1-2 minutes to remove the membrane transfer liquid on the membrane.
7. It is blocked by slowly shaking on a shaker at room temperature for 45 minutes.
8. It is added with a diluted primary antibody and incubated at room temperature for one hour with slow shaking.
9. It is added with the washing liquid, and slowly shaken for washing on the shaker for 5 minutes for 3 times in total.
10. It is added with a diluted secondary antibody and incubated at room temperature for one hour with slow shaking.
11. It is added with the washing liquid, and slowly shaken for washing on the shaker for 5 minutes for 3 times in total.
12. Chemiluminescence detection.
13. The Western Blotting result picture is quantitatively analyzed with software Image J.

Table 2: Relative Expression Quantity of Protein Before and After Optimization (As Detected by Western Blotting)

| | GFP (relative expression quantity $\pm$ standard deviation) | JNK3 (relative expression quantity $\pm$ standard deviation) |
|---|---|---|
| **After Optimization** | 22.06 $\pm$ 1.78 | 8.01 $\pm$ 0.21 |
| **Wild Type** | 1.19 $\pm$ 0.16 | 1.09 $\pm$ 0.10 |
| **Ratio** | 18.37 $\pm$ 2.90 | 7.42 $\pm$ 0.58 |

*Relative expression quantity: a protein expression quantity divided by the minimum value of expression quantity in three replicated experiments of wild-type sequences

As can be seen from Table 2, the expression quantities of JNK3 and GFP proteins after being subjected to the three-step hybrid codon optimization of this patent are respectively increased by 7.42 $\pm$ 0.58 times and 18.37 $\pm$ 2.90 times compared with that of the wild-type sequence, which fully proves the high efficiency of the new algorithm. In the actual production of a company, we also compare and test the optimization effects of this algorithm and other algorithms on multiple proteins, which also proves that this algorithm is more stable and efficient.
The parts not involved in the present invention are all the same as those in the prior art or can be realized by using the prior art.

**Claims**

1. A computer-implemented codon optimization method for the expression sequence of a protein based on an immune algorithm,

   wherein the codon optimization method comprises the following three steps: a first step of local optimization, that is, cleaving the protein sequence into nonoverlapping sequence fragments $A_1, A_2...A_n$, and then using the immune algorithm to complete the codon optimization for each sequence fragment, so as to generate an approximately optimal DNA sequence set $B_1, B_2...B_n$; a second step of global optimization, that is, initializing the DNA coding sequence of the full length of the protein based on $B_1, B_2...B_n$ utilizing a genetic algorithm, and screening out the optimal DNA sequence $C_1$ of the protein sequence; and a third step of fine adjustment and optimization, which comprises performing exhaustive optimization on the 5' terminal of the DNA sequence corresponding to the N-terminal region of the encoded protein to generate a DNA sequence $C_2$, and eliminating an expression inhibitory motif, to finally generate the optimal expression sequence $D$ of the protein;

wherein the immune genetic algorithm adopts a multi-objective optimization method to perform local optimization on the protein fragments, the population initialization is based on a duplex codon table of a sequence encoding a highly-expressed protein, and each gene is directly encoded by synonymous codons; and in the optimization process, antibody diversity is ensured and the phenomenon of population degeneration is prevented by calculating antibody information entropy, antibody population similarity, antibody concentration and polymerization fitness of the immune genetic algorithm and updating memory cells, so as to increase the global search capability of the algorithm;

wherein the genetic algorithm adopts the multi-objective optimization method to perform global optimization on the full sequence of the protein, an initialized population is randomly generated based on optimized fragments subjected to local optimization, and each gene is directly encoded by an optimized sequence set of each protein fragment;

wherein the fine adjustment and optimization uses the exhaustive method to calculate and sort the minimum free energy MFE, Codon Context and CAI at the 5' terminal of the DNA sequence, and selects the optimum coding sequence for the N-terminal of the protein sequence according to the sorting result.

2. The codon optimization method according to claim 1, wherein the protein is a compound consisting of more than 20 amino acids; the protein is a secretory protein, a membrane protein, a cytoplasmic protein or a nuclear protein in terms of localization; the protein is an antibody protein, a regulatory protein or a structural protein in terms of function; the protein is a homologously expressed protein or a heterologously expressed protein in terms of source; the protein is a natural protein or an artificially-modified protein, a complete protein/antibody or a truncated partial protein/antibody, or a fusion protein formed from 2 or more proteins or from a protein and a peptide chain in terms of sequence; optionally wherein the antibody is selected from an intact antibody, Fab, ScFV, SdAb, a chimeric antibody, a bispecific antibody, and a Fc fusion protein.

3. The codon optimization method according to claim 1, wherein the codon optimization method is applicable to at least one of the following host expression systems: 1) a mammalian expression system; 2) an insect expression system; 3) a yeast expression system; 4) a *Escherichia coli expression* system; 5) a *Bacillus subtilis* expression system; 6) a plant expression system, and 7) a cell-free expression system.

4. The codon optimization method according to claim 1, wherein the codon optimization method is applicable to at least one of the following expression vectors: a transient expression vector, a stable expression vector, a viral expression vector, a non-viral expression vector, induced and non-induced expression vectors.

**Patentansprüche**

1. Computerimplementiertes Codon-Optimierungsverfahren für die Expressionssequenz eines Proteins basierend auf einem Immunalgorithmus,

wobei das Codon-Optimierungsverfahren die folgenden drei Schritte umfasst: einen ersten Schritt der lokalen Optimierung der Proteinsequenz, also des Spaltens derselben in nicht überlappende Sequenzfragmente $A_1$, $A_2$ ... $A_n$, und dann des Verwendens des Immunalgorithmus, um die Codon-Optimierung für jedes Sequenzfragment abzuschließen, um so einen annähernd optimalen DNA-Sequenzsatz $B_1$, $B_2$ ... $B_n$ zu erzeugen; einen zweiten Schritt der globalen Optimierung der Proteinsequenz, also dem Initialisieren der DNA-Kodierungssequenz der gesamten Länge des Proteins basierend auf $B_1$, $B_2$ ... $B_n$ unter Verwendung eines genetischen Algorithmus und dem Aussortieren der optimalen DNA-Sequenz $C_1$; und einen dritten Schritt der Feinanpassung und Optimierung, umfassend das Durchführen einer umfassenden Optimierung am 5'-Terminus der DNA-Sequenz umfasst, die der N-terminalen Region des kodierten Proteins entspricht, um eine DNA-Sequenz $C_2$ zu erzeugen, und das Eliminieren eines expressionshemmenden Motivs, um schließlich die optimale Expressionssequenz $D$ des Proteins zu erzeugen;

wobei der immungenetische Algorithmus ein Mehrzieloptimierungsverfahren anwendet, um eine lokale Optimierung der Proteinfragmente durchzuführen, die Populationsinitialisierung auf einer Duplex-Codon-Tabelle einer Sequenz basiert, die für ein stark exprimiertes Protein codiert, und für jedes Gen direkt durch synonyme Codons kodiert wird; und im Optimierungsprozess Antikörpervielfalt sichergestellt wird und das Phänomen der Populationsdegeneration verhindert wird, indem Antikörperinformationsentropie, Antikörperpopulationsähnlichkeit, Antikörperkonzentration und Polymerisationseignung des immungenetischen Algorithmus berechnet und Gedächtniszellen aktualisiert werden, um die globale Suchfähigkeiten des Algorithmus zu verbessern;

wobei der genetische Algorithmus das Mehrzieloptimierungsverfahren anwendet, um eine globale Optimierung für die gesamte Sequenz des Proteins durchzuführen, eine initialisierte Population zufällig auf der Grundlage optimierter Fragmente generiert wird, die einer lokalen Optimierung ausgesetzt werden, und für jedes Gen durch einen optimierten Sequenzsatz für jedes Proteinfragment direkt kodiert wird;

wobei die Feinanpassung und Optimierung das umfassende Verfahren verwendet, um die minimale freie Energie MFE, den Codon-Kontext und den CAI am 5'-Terminus der DNA-Sequenz zu berechnen und zu sortieren und die optimale Codierungssequenz für den N-Terminus der Proteinsequenz entsprechend dem Sortierergebnis auszuwählen.

2. Verfahren zur Codon-Optimierung nach Anspruch 1, wobei das Protein eine Verbindung ist, die aus mehr als 20 Aminosäuren besteht; das Protein hinsichtlich der Lokalisierung ein sekretorisches Protein, ein Membranprotein, ein cytoplasmatisches Protein oder ein Kernprotein ist; das Protein hinsichtlich seiner Funktion ein Antikörperprotein, ein regulatorisches Protein oder ein Strukturprotein ist; das Protein hinsichtlich der Quelle ein homolog exprimiertes Protein oder ein heterolog exprimiertes Protein ist; das Protein hinsichtlich der Sequenz ein natürliches Protein oder ein künstlich verändertes Protein, ein vollständiges Protein / ein vollständiger Antikörper oder ein verkürztes partielles Protein / ein verkürzter partieller Antikörper oder ein Fusionsprotein, ausgebildet aus 2 oder mehr Proteinen und einer Peptidkette, ist; optional wobei der Antikörper ausgewählt ist aus einem intakten Antikörper, Fab, ScFV, SdAb, einem chimären Antikörper, einem bispezifischen Antikörper und einem Fc-Fusionsprotein.

3. Codon-Optimierungsverfahren nach Anspruch 1, wobei das Codon-Optimierungsverfahren auf mindestens eines der folgenden Wirtsexpressionssysteme anwendbar ist: 1) ein Säugerexpressionssystem; 2) ein Insektenexpressionssystem; 3) ein Hefeexpressionssystem; 4) ein Escherichia-coli-Expressionssystem; 5) ein *Bacillus-subtilis-Expressionssystem*; 6) ein pflanzliches Expressionssystem und 7) ein zellfreies Expressionssystem.

4. Codon-Optimierungsverfahren nach Anspruch 1, wobei das Codon-Optimierungsverfahren auf mindestens einen der folgenden Expressionsvektoren anwendbar ist: einen transienten Expressionsvektor, einen stabilen Expressionsvektor, einen viralen Expressionsvektor, einen nichtviralen Expressionsvektor, induzierte und nichtinduzierte Expressionsvektoren.

**Revendications**

1. Procédé d'optimisation des codons mis en oeuvre par ordinateur pour la séquence d'expression d'une protéine basé sur un algorithme immunitaire,

le procédé d'optimisation des codons comprenant les trois étapes suivantes : une première étape d'optimisation locale, à savoir, clivage de la séquence de protéine en fragments de séquence non chevauchants $A_1, A_2...A_n$, puis utilisation de l'algorithme immunitaire pour terminer l'optimisation des codons pour chaque fragment de séquence, de façon à générer un ensemble de séquences d'ADN approximativement optimal $B_1, B_2...B_n$, une deuxième étape d'optimisation globale, à savoir, l'initialisation de la séquence codante d'ADN de la pleine longueur de la protéine sur la base de $B_1, B_2...B_n$ au moyen d'un algorithme génétique, et le criblage de la séquence d'ADN optimale $C_1$ de la séquence de protéine ; et une troisième étape d'ajustement fin et d'optimisation, qui comprend la conduite d'une optimisation exhaustive sur l'extrémité 5' de la séquence d'ADN correspondant à la région N-terminale de la protéine codée pour générer une séquence d'ADN $C_2$, et l'élimination d'un motif inhibiteur d'expression, pour générer finalement la séquence d'expression optimale $D$ de la protéine ;

dans lequel l'algorithme génétique immunitaire adopte un procédé d'optimisation à objectifs multiples pour effectuer une optimisation locale sur les fragments de protéine, l'initialisation de la population est basée sur une table de codons duplex d'une séquence codant pour une protéine hautement exprimée, et chaque gène est directement codé par des codons synonymes ; et dans le processus d'optimisation, la diversité d'anticorps est assurée et le phénomène de dégénérescence de population est évité par calcul d'entropie d'informations d'anticorps, de similarité de population d'anticorps, concentration d'anticorps et justesse de polymérisation de l'algorithme génétique immunitaire et mise à jour des cellules de mémoire, de façon à augmenter la capacité de recherche globale de l'algorithme ;

dans lequel l'algorithme génétique adopte le procédé d'optimisation à objectifs multiples pour effectuer une optimisation globale sur la séquence totale de la protéine, une population initialisée est générée de façon aléatoire sur la base de fragments optimisés soumis à une optimisation locale, et chaque gène est directement codé par un ensemble de séquences optimisé de chaque fragment de protéine ;

dans lequel l'ajustement fin et l'optimisation utilisent le procédé exhaustif pour calculer et trier l'énergie libre minimale MFE, le contexte de codons et CAI à l'extrémité 5' de la séquence d'ADN, et sélectionne la séquence codante optimale pour l'extrémité N-terminale de la séquence de protéine selon le résultat du tri.

2. Procédé d'optimisation des codons selon la revendication 1, dans lequel la protéine est un composé constitué de plus de 20 acides aminés ; la protéine est une protéine sécrétoire, une protéine membranaire, une protéine cytoplasmique ou une protéine nucléaire en termes de localisation ; la protéine est une protéine d'anticorps, une protéine régulatrice ou une protéine structurale en termes de fonction ; la protéine est une protéine exprimée de façon homologue ou une protéine exprimée de façon hétérologue en termes de source ; la protéine est une protéine naturelle ou une protéine modifiée de façon artificielle, une protéine/un anticorps complet ou une protéine/un anticorps partiel tronqué, ou une protéine de fusion formée de 2 protéines ou plus parmi une protéine et une chaîne peptidique en termes de séquence ; éventuellement dans lequel l'anticorps est choisi parmi un anticorps intact, Fab, ScFV, SdAb, un anticorps chimérique, un anticorps bispécifique et une protéine de fusion de Fc.

3. Procédé d'optimisation des codons selon la revendication 1, le procédé d'optimisation des codons étant applicable à au moins l'un des systèmes d'expression d'hôte suivants : 1) un système d'expression de mammifère ; 2) un système d'expression d'insecte ; 3) un système d'expression de levure ; 4) un système d'expression *d'Escherichia coli* ; 5) un système d'expression de *Bacillus subtilis* ; 6) un système d'expression de plante, et 7) un système d'expression acellulaire.

4. Procédé d'optimisation des codons selon la revendication 1, le procédé d'optimisation des codons étant applicable à au moins l'un des vecteurs d'expression suivants : un vecteur d'expression transitoire, un vecteur d'expression stable, un vecteur d'expression viral, un vecteur d'expression non viral, des vecteurs d'expression induits et non induits.

Slicing the protein sequence into non-overlapping sequence fragments with a sliding window

Protein sequence

Protein fragment $A_1$ — Immune algorithm →

Protein fragment $A_2$ — Immune algorithm →

Protein fragment $A_3$ — Immune algorithm →

⋮

Protein fragment $A_n$ — Immune algorithm →

(Approximate) Optimal DNA Sequence Set $B_1$ of $A_1$

(Approximate) Optimal DNA Sequence Set $B_2$ of $A_2$

(Approximate) Optimal DNA Sequence Set $B_3$ of $A_3$

⋮

(Approximate) Optimal DNA Sequence Set $B_n$ of $A_n$

optimal DNA sequence $D$ for a full sequence of protein ← eliminating inhibitory Motif — The optimal DNA sequence $C_2$ of the full sequence of protein ← Secondary optimization of the 5' terminal of the DNA sequence — The optimal DNA sequence $C_1$ of the full sequence of protein ← Genetic algorithm

# FIG. 1

Parameter setting (population size N, memory cell antibody K, crossover rate C, mutation rate M, similarity threshold S₀)

Yes      Initial response?      No

Randomly generating all N initial antibodies based on a duplex codon frequency table

Parent antibodies plus K antibodies of the memory cells

Calculating antibody fitness (calling Codon Context and CAI calculation functions)

Producing new antibodies (fitness-based multi-target antibody selection [total number N of antibodies], crossover, variation)

Calculating antibody population similarity S (calling an information entropy calculation function)

No      Similarity S>S₀?

**gen = gen + 1**
Updating the memory cells      Yes

Randomly generating an initial antibody P based on the duplex codon frequency table, where the total number of antibodies is (P+N)

Calculating an antibody concentration and a polymerization fitness

Producing a new antibody (fitness-based multi-target antibody selection [total number N of antibodies] and update of the memory cells)

No      Has the termination condition been met?

**gen = gen + 1**      Yes

Outputting an optimized sequence

# FIG. 2

Parameter setting (population size N, crossover rate C, mutation rate M)

Generating all initial antibodies by randomly combining optimized sets
of protein fragments

Calculate individual fitness (calling Codon Context and CAI calculation functions)

Multi-objective individual selection based on the fitness

Crossover and Variation

Generating a new population and recording optimized individuals

No

**gen = gen + 1**

Has the termination condition
been met?

Yes

Outputting the optimal sequence in history

# FIG. 3

50 bp DNA of an expression vector located upstream of the initiation codon of the protein sequence + all possible DNA sequences encoding the N terminal of the protein sequence

all possible DNA sequences encoding the N terminal of the protein sequence

Calculating minimum free energy

Calculating Codon Context and CAI

Sorting the DNA sequences encoding the N terminal of the protein sequence according to the minimum free energy, Codon Context and CAI

Optimal 5'-terminal sequence

# FIG. 4

| | 1~1242 bp | 1243~1266 bp |
|---|---|---|
| Before optimization: | U34820.1 | Flag Tag |
| JNK3 | | |
| After optimization: | The optimized DNA sequence | Flag Tag |

| | 1~3048 bp | 3049~3072 bp |
|---|---|---|
| Before optimization: | AY174111.1 | Flag Tag |
| GFP | | |
| After optimization: | The optimized DNA sequence | Flag Tag |

# FIG. 5

**FIG. 6**

**FIG. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014244228 A1

**Non-patent literature cited in the description**

- **JOSHUA B. PLOTKIN ; GRZEGORZ KUDLA.** *Nature Reviews Genetics,* 2011
- **EVELINA ANGOV.** *Biotechnology Journal,* 2011
- **NATHAN GOULD et al.** *Frontiers in Bioengineering and Biotechnology,* 2014